# FASCICULE DE BREVET EUROPEEN

(11) **EP 3 611 660 B1**
(45) Date de publication et mention de la délivrance du brevet: **09.03.2022**
(21) Numéro de dépôt: 19306017.5
(22) Date de dépôt: 14.08.2019
(51) Int. Cl.: G06K 9/00, G01J 5/34

(54) **PROCÉDÉ DE FABRICATION D'UNE MATRICE DE PIXELS D'UN CAPTEUR DE MOTIF THERMIQUE ET CAPTEUR ASSOCIÉ**
ERZEUGUNGSVERFAHREN EINER PIXELMATRIX EINES THERMISCHEN MUSTERSENSORS, UND ENTSPRECHENDER SENSOR
METHOD OF MANUFACTURING AN ARRAY OF PIXELS FROM A THERMAL PATTERN SENSOR AND ASSOCIATED SENSOR

(30) Priorité: 16.08.2018 FR 1857511
(43) Date de publication de la demande: 19.02.2020
(73) Titulaire: Idemia Identity & Security France, 92400 Courbevoie (FR); Commissariat à l'Energie Atomique et aux Energies Alternatives, 75015 Paris (FR)
(72) Inventeur: MAINGUET, Jean-François, 38054 GRENOBLE CEDEX 09 (FR); FOURRE, Joël-Yann, 92400 COURBEVOIE (FR); SERBUTOVIEZ, Christophe, 38054 GRENOBLE CEDEX 09 (FR); BENWADIH, Mohammed, 38054 GRENOBLE CEDEX 09 (FR)
(74) Mandataire: Regimbeau

(56) Documents cités:
- WO-A1-2018/020176
- XIAOYU DING ET AL: "A review of the operating limits in slot die coating processes", AICHE JOURNAL, vol. 62, no. 7, 4 mai 2016 (2016-05-04), pages 2508-2524, XP055591404, US ISSN: 0001-1541, DOI: 10.1002/aic.15268

## Description

### DOMAINE TECHNIQUE GENERAL

L'invention concerne un procédé de fabrication d'un capteur de motif thermique, par exemple un capteur d'empreintes digitales dit « actif », réalisant une détection thermique active ou passive et comprenant, de préférence, une matrice active ou passive de pixels. L'invention concerne plus particulièrement la fabrication de la matrice de pixels.

### ETAT DE LA TECHNIQUE

Un capteur d'empreintes digitales comporte des moyens de détection thermique. Ces moyens de détection thermique peuvent correspondre à des éléments pyroélectriques, des diodes, des thermistances ou tout autre élément sensible à la température permettant de convertir une variation de température en une variation de potentiel ou de courant électrique.

On connaît des documents US 6 091 837, EP 2 385 486 A1 et WO 2018/020176 des capteurs d'empreintes digitales dits « actifs » réalisant une détection thermique active et comprenant une matrice passive de pixels.

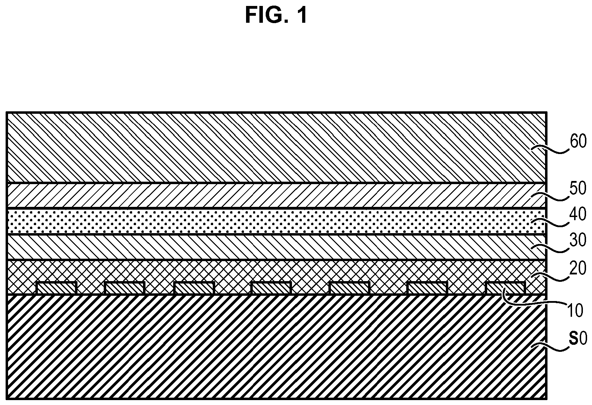

La **figure 1** illustre une vue en coupe la structure d'un tel capteur qui comprend, notamment, les couches ci-dessous déposées les unes après les autres au-dessus d'un support S0.

Un tel support S0 (par exemple un substrat) est en PET ou PEN ou PI, ou encore de type CIU (ABF, FR4, ...) ou encore en verre.

La première couche 10 est constituée par un motif en matériau conducteur électrique (Au, Ag, Al, Cu) comportant des colonnes M1 métalliques et une ligne de masse (non représentée).

La deuxième couche 20 est constituée d'un matériau pyroélectrique composé d'un copolymère de PVDF (en anglais, « PolyVinyliDeneFluoride » pour Polyfluorure de Vinydilène) et de Trfe (trifluoroethylène) qui recouvre les lignes M1.

La troisième couche 30 est en matériau conducteur électrique et supporte une quatrième couche 40 en matériau diélectrique elle-même supportant une cinquième couche 50 comportant des lignes en matériau conducteur électrique, de préférence métallique, qui vont être chauffées.

Enfin une sixième couche 60 de protection recouvre l'ensemble.

Chaque pixel est formé par une capacité pyroélectrique elle-même formée de deux électrodes conductrices électriquement et thermiquement (mais dans une moindre mesure afin d'éviter un éventuel court-circuit thermique latéral) (première et troisième couches 10, 30) entre lesquelles une portion de matériau pyroélectrique est disposée, et un élément chauffant (couche 50). Cet élément chauffant dissipe une certaine quantité de chaleur dans le pixel, et l'échauffement du pixel est mesuré au bout d'un certain temps d'acquisition, appelé temps d'intégration, en présence du doigt sur le capteur. Cela permet de distinguer, au niveau de chaque pixel, la présence d'une crête ou d'une vallée de l'empreinte détectée suivant que la chaleur est absorbée par la peau (pixel en présence d'une crête de l'empreinte) ou conservée dans le pixel (pixel en présence d'une vallée de l'empreinte). Cela conduit à une température finale plus faible dans le cas d'un pixel en présence d'une crête, où la chaleur est absorbée par la peau, par rapport à un pixel en présence d'une vallée.

De manière classique un tel capteur peut être fabriqué au moyen de diverses techniques d'impression ou par des méthodes soustractives comme la photolithographie notamment pour ce qui est de l'obtention de la matrice passive de pixels. Toutes les techniques d'impression ne permettent pas d'accéder à ce dispositif avec le même coût. Aussi, il existe un besoin de pouvoir mettre en oeuvre des techniques de dépôt par voie liquide (en anglais, « slot die ») qui sont peu coûteuses pour fabriquer un tel capteur.

### PRESENTATION DE L'INVENTION

Un but de l'invention est de proposer une matrice de pixels d'un capteur de motif thermique, qui est un motif tridimensionnel (3D), pouvant être obtenue au moyen d'un procédé à rouleau (en anglais « roll to roll ») ou feuille à feuilles (en anglais, « sheet to sheet ») combiné à des techniques d'impression / dépôt des couches qui soient peu coûteuses.

A cet effet, l'invention propose selon un premier aspect un procédé de fabrication d'une matrice de pixels d'un capteur de motif thermique, le procédé comprenant les étapes de :
fourniture d'un substrat ;
formation d'une première couche en matériau électriquement conducteur, comprenant :
   o un dépôt de pistes électriquement conductrices s'étendant selon une première direction d'allongement ;
   o un dépôt de pattes de connexion, s'étendant depuis les pistes, selon une deuxième direction d'allongement, différente de la première direction d'allongement, et en se dirigeant vers un premier bord du substrat, dit bord de sortie ;
   o un dépôt d'une bande de masse destinée à former la masse du capteur ;
   formation d'une deuxième couche en matériau pyroélectrique recouvrant les pistes et laissant libre au moins une partie des pattes de connexion ;
   formation d'une troisième couche en matériau électriquement conducteur déposée à cheval entre la couche de matériau pyroélectrique et la bande de masse sans recouvrir la partie libre des pattes de connexion ;
   formation d'une quatrième couche en matériau diélectrique en contact avec la troisième couche et s'étendant de manière à recouvrir les pistes de la première couche ;
   dépôt d'une cinquième couche comprenant des pistes électriquement conductrices chauffantes lesdites pistes électriquement conductrices chauffantes s'étendant depuis la bande de masse vers le premier bord du substrat de manière à recouvrir les pistes de la première couche ;
   dépôt d'une sixième couche de protection ;
   procédé de fabrication dans lequel le dépôt de la deuxième couche et/ou le dépôt de la troisième couche et/ou le dépôt de la quatrième couche et/ou le dépôt de la sixième couche est mis en oeuvre par dépôt par voie liquide au moyen d'une filière à fente.

Les avantages de l'invention sont multiples.

L'agencement des différentes couches de la matrice de pixels permet de mettre en oeuvre des techniques simples et peu coûteuses pour fabriquer lesdites couches.

En effet, les couches constituées d'au moins une bande de largeur préférentiellement supérieure ou égale à 5 mm peuvent être déposées par voie liquide au moyen d'une technique mettant en oeuvre une filière en forme de fente au travers de laquelle le matériau à déposer est amené sur la surface sous forme d'encre. En anglais, cette technique est connue sous le terme « slot die » ou « slit coating ». Cette technique est très adaptée lorsqu'il s'agit de déposer des bandes continues ou de longues bandes.

L'invention est avantageusement complétée par les caractéristiques suivantes, prises seules ou en une quelconque de leurs combinaisons techniquement possibles :
- les couches sont formées successivement de la manière suivante : formation de la première couche, formation de la deuxième couche, formation de la troisième couche, formation de la quatrième couche, formation de la cinquième couche, formation de la sixième couche ;
- la première couche comprend en outre une bande de polarisation connectée à des colonnes du motif métallique de la première couche ;
- les pistes de la première couche forment un serpentin, les pistes étant connectées deux à deux au moyen de bandelettes métalliques ;
- la première couche comprend en outre des pavés métalliques destinés à être dans le prolongement des pistes électriquement conductrices chauffantes de la cinquième couche ;
- les pistes et les pattes de connexions de la première couche forment une pluralité de L imbriqués les uns dans les autres ;
- à l'issue du procédé, les pattes de connexions sont toutes en-dessous des pistes électriquement conductrices chauffantes de la cinquième couche ;
- les pistes et les pattes de connexion de la première couche forment une alternance de L et de L retournés ;
- l'étape de formation de la troisième couche est mise en oeuvre de sorte à ménager un espace selon la deuxième direction d'allongement le long duquel la deuxième couche en matériau pyroélectrique reste apparente ;
- au cours de l'étape de formation de la deuxième couche, on réalise une juxtaposition de plusieurs bandes de matériau pyroélectrique afin de réaliser plusieurs matrices de pixels en parallèle.

L'invention se rapporte selon un deuxième aspect à une matrice de pixels d'un capteur de motif thermique fabriquée par un procédé tel que défini ci-avant.

L'invention concerne selon un troisième aspect un capteur de motif thermique comprenant une matrice de pixels telle que définie ci-avant, la matrice étant typiquement passive ou active.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels, outre la figure 1 déjà discutée :
- la figure 2a illustre une vue de dessus d'une matrice de pixels selon un premier mode de réalisation de l'invention ;
- la figure 2b illustre une vue de côté en coupe de la matrice de la figure 2a ;
- les figures 3a à 8b illustrent le dépôt des couches successives au cours d'un procédé de fabrication d'une matrice de pixels selon l'invention.
- la figure 9 illustre une vue de dessus d'une matrice de pixels selon un deuxième mode de réalisation ;
- la figure 10 illustre une vue de dessus d'une matrice de pixels selon un mode de réalisation complémentaire au premier mode de réalisation ;
- la figure 11 illustre une vue dessus d'une matrice de pixels selon un mode de réalisation complémentaire au premier mode de réalisation ;
- la figure 12 illustre une vue de dessus d'une matrice de pixels selon un mode de réalisation complémentaire au premier mode de réalisation ou au second mode de réalisation ;
- la figure 13 illustre une vue de dessus d'une matrice de pixels selon un mode complémentaire mode de réalisation de la figure 12.

Sur l'ensemble des figures les éléments similaires portent des références identiques.

### DESCRIPTION DETAILLEE DE L'INVENTION

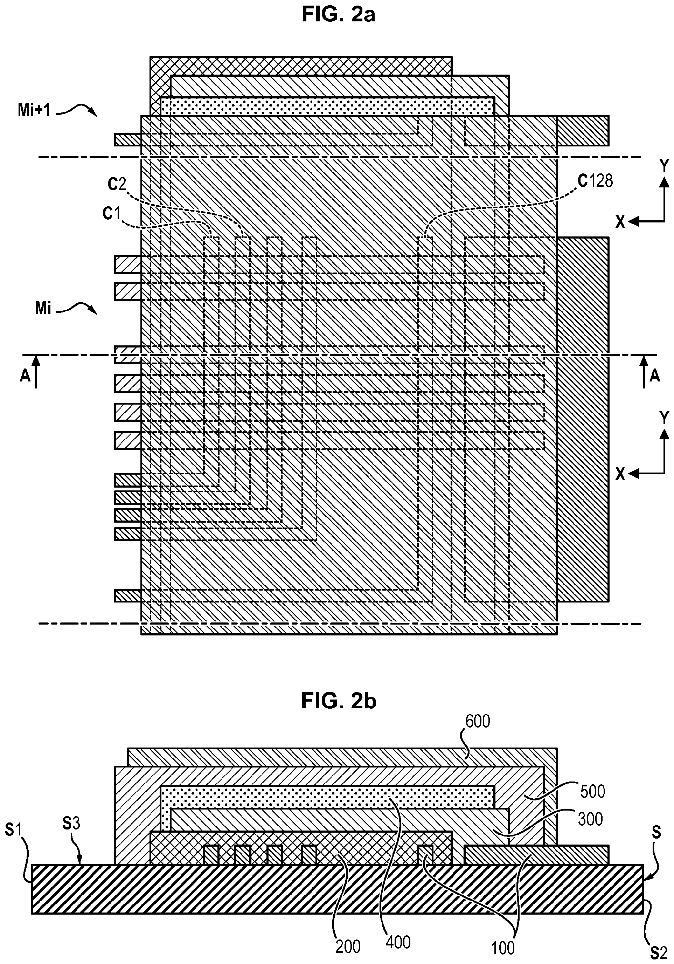

La **figure 2a** illustre une vue de dessus d'une matrice d'un capteur thermique selon un mode de réalisation de l'invention et la **figure 2b** illustre une vue de côté d'une coupe A-A du motif de la figure 2a.

Sur cette figure, on voit un motif complet Mi et le début du motif suivant Mᵢ₊₁, les deux étant séparés sur la figure par un trait épais en pointillés (il s'agit en fait d'une ligne imaginaire de découpe selon laquelle à l'issue de toutes les étapes de fabrication la matrice sera découpée selon cette ligne).

En effet, selon un mode de réalisation préféré, et dans ce qui suit, on se place dans le cas d'une impression à rouleaux (en anglais « roll to roll ») selon laquelle un rouleau comprenant un flux continu de supports est déroulé pour être amené à plusieurs étages d'impression. L'intérêt de l'impression à rouleau est qu'elle est plus rapide qu'une impression support par support et évite les opérations de manipulation pour amener un support d'un étage d'impression à un autre étage d'impression.

En outre, l'impression à rouleaux permet de manipuler des supports très minces. En effet la manipulation support par support exige une certaine rigidité alors qu'en rouleau, la tension du rouleau rend des supports très minces manipulables.

On comprendra que plusieurs matrices peuvent êtres obtenues sur la largeur d'un seul rouleau, la largeur étant mesurée selon une direction sensiblement perpendiculaire à une direction de déroulement du rouleau. Le rouleau de support doit alors présenter une largeur suffisante pour réaliser une juxtaposition de plusieurs bandes.

Bien entendu, on comprend que la matrice peut être obtenue au moyen d'un procédé d'impression ou de dépôt par voie liquide en mode feuille à feuille.

En outre, on va décrire ci-après la fabrication d'une matrice.

Toutefois,comme indiqué ci-avant, plusieurs matrices peuvent être obtenues en parallèle sur la largeur du support, conditionnellement à la largeur des rouleaux sur lesquels est enroulé le support. En relation avec les **figures 2a** et **2b**, la matrice comprend un empilement de plusieurs couches 100, 200, 300, 400, 500, 600 sur un support S tel qu'un substrat.

Cet empilement n'est pas limitatif comme on le verra par la suite.

Le support S présente un premier bord s1, un deuxième bord s2 et une surface s3 supérieure rectangulaire ayant une largeur selon un axe X et une longueur selon un axe Y.

Le support S est par exemple en verre ou en matériau semiconducteur (tel que du Si). En outre, le support S présente une épaisseur comprise entre 25 et 200 microns, typiquement 50 à 125 microns.

De préférence, et afin d'être compatible avec un procédé d'impression ou de dépôt par voie liquide par rouleau, le support S est un matériau souple par exemple à base de PI (polyimide) ou de PEN (polytéthylène naphtalate) ou de PET (polyéthylène téréphtalate).

Sur ce support S plusieurs couches sont déposées, typiquement par des techniques de dépôt en voie liquide qui seront décrites plus loin.

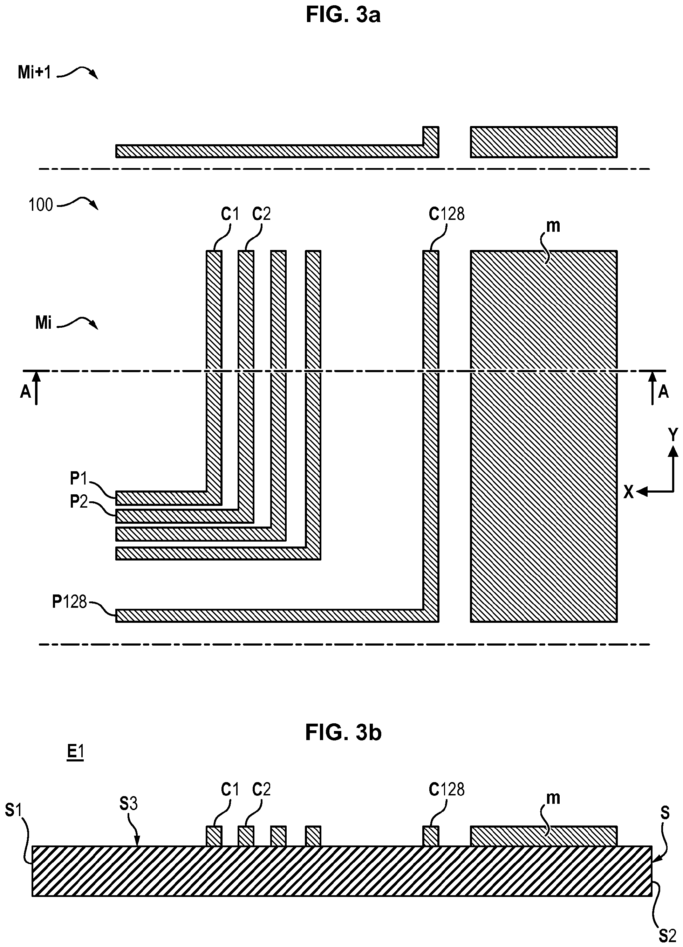

Une première couche 100 comprend un motif conducteur électrique (voir les **figures 3a** et **3b**), le motif métallique conducteur électrique comprenant
- Des pistes formant des colonnes c₁, c₂, c₁₂₈ de la matrice de pixels et qui constituent l'électrode inférieure. Ces pistes sont parallèles entre elles selon une première direction d'allongement (selon l'axe Y). Typiquement la matrice comprend 128 pistes.
- Des pattes p₁, p₂, p₁₂₈ de connexion, s'étendant depuis les pistes, selon une deuxième direction d'allongement (selon l'axe X), et en se dirigeant vers un premier bord du substrat S dit bord de sortie (c'est à-dire le bord par lequel les connexions/soudures seront effectuées).
- une bande m de masse parallèle aux pistes et s'étendant le long d'un second bord opposé au premier bord (selon l'axe Y) ;

Le motif conducteur électrique est en matériau métallique, typiquement de l'or, ou un métal moins précieux (Argent, Aluminium ou Cuivre, etc.). L'épaisseur du motif métallique est comprise entre 50 et 200 nm.

A noter que la figure 2b est une coupe selon l'axe AA de sorte que les pattes de connexions ne sont pas visibles.

Cette première couche est avantageusement déposée au moyen d'une impression par offset ou par gravure ou héliogravure ou bien encore par lithographie ou structuration laser. On remarquera que sont déposées des bandes de largeur constante.

La technique par offset consiste en ce qu'un cylindre vient déposer le métal sur un second cylindre qui passe sur le support S. Bien entendu le diamètre du rouleau doit être correctement agencé pour faire des motifs réguliers. Cette technique d'impression présente l'avantage d'imprimer des motifs précis.

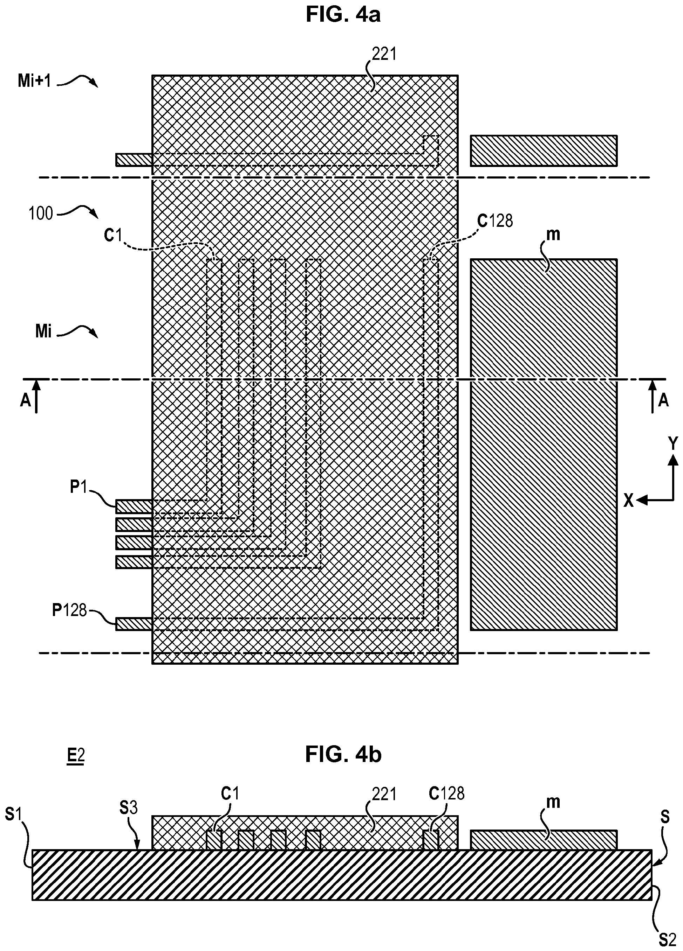

Une deuxième couche 200 (voir les **figures 4a** et **4b**), comprenant, de préférence une bande 221 rectangulaire, en matériau pyroélectrique recouvrant les pistes c₁, c₂, c₁₂₈ et laissant libre au moins une partie des pattes p₁, p₂, p₁₂₈ de connexion.

Un matériau pyroélectrique est par exemple du P(VDF-TrFE) ou du PVDF. D'autres variantes peuvent être envisagées pour le choix du matériau dès lors qu'il est adapté pour former une capacité pyroélectrique.

Comme visible sur la figure 4a, la bande 221 est de largeur constante (rectangulaire sur la figure 4a) et recouvre les pistes c₁, c₂, c₁₂₈ mais pas l'intégralité des pattes p₁, p₂, p₁₂₈ de connexion qui sont laissées apparentes pour pouvoir effectuer des connexions/soudures. De même la bande 221 en matériau pyroélectrique ne recouvre pas (même partiellement) la bande m de masse. Ainsi, éventuellement il y a un espace entre la bande 221 en matériau pyroélectrique et la bande m rectangulaire de masse.

A titre d'exemple, l'épaisseur de la deuxième couche 200 (matériau pyroélectrique) est comprise entre 1 et 10 microns de préférence de 2 à 5 microns et encore plus préférentiellement de 3 microns.

Cette deuxième couche 200 est avantageusement déposée par voie liquide au moyen d'une filière à fente (« slot die ») réalisant une bande continue dans la direction de déroulement du rouleau, ou en réalisant de longues bandes dans le cas d'un mode feuille à feuille, ou encore une juxtaposition de plusieurs bandes lorsque plusieurs matrices sont fabriquées en parallèle. De préférence, au cours de l'étape de dépôt les bandes s'étendent entre chaque ligne de découpe, la forme rectangulaire étant obtenue après découpe de la matrice.

Optionnellement, la couche de matériau pyroélectrique peut être déposée en deux (ou plus) passes, ou deux couches successives pour faciliter et obtenir un dépôt plus homogène, surtout lorsque l'épaisseur finale devient importante, par exemple 5 microns.

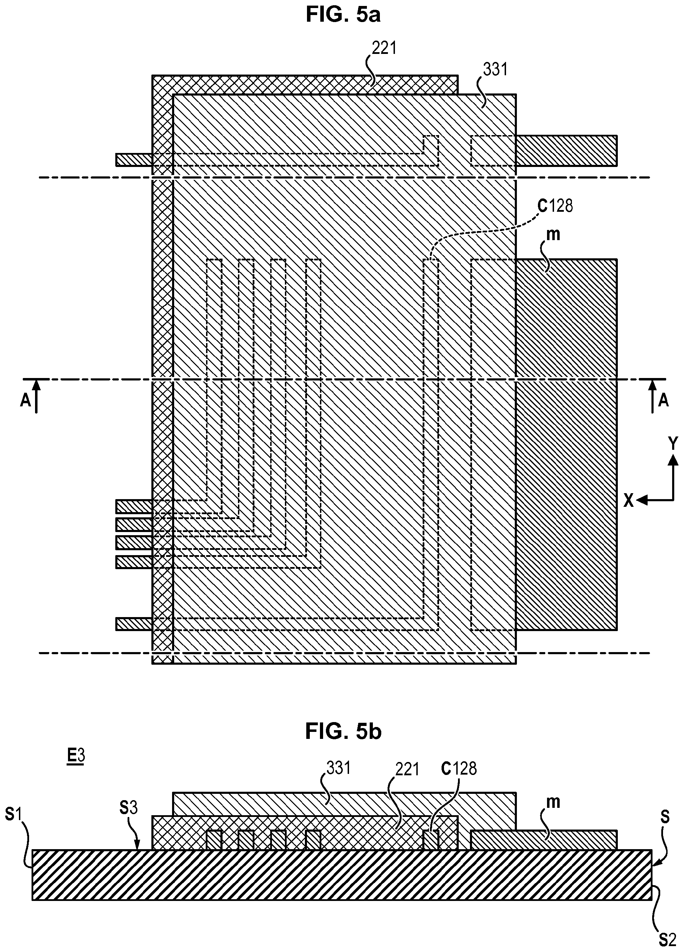

Une troisième couche 300 (voir les **figures 5a** et **5b**) comprenant une bande 331 de largeur constante en matériau conducteur électrique à cheval entre la bande 221 en matériau pyroélectrique de la deuxième couche et la bande m de masse sans recouvrir complétement la partie libre des pattes de connexions de la première couche. Cette troisième couche est avantageusement déposée par voie liquide au moyen d'une filière à fente (« slot die ») réalisant une bande continue dans la direction de déroulement du rouleau ou en réalisant de longues bandes dans le cas d'un mode feuille à feuille ou encore une juxtaposition de plusieurs bandes lorsque plusieurs matrices sont fabriquées en parallèle. De préférence, au cours de l'étape de dépôt les bandes s'étendent entre chaque ligne de découpe, la forme rectangulaire étant obtenue après découpe de la matrice. Cette troisième couche 300 constitue une électrode supérieure pour la matrice du capteur et est typiquement en PEDOT:PSS ou en encre conductrice à base d'argent.

Comme visible la figure 5a, la bande 331 de la troisième couche 300 est connectée à la bande m de masse. Là encore les pattes p₁, p₂, p₁₂₈ de connexions sont laissées apparentes.

De préférence, une partie de la bande rectangulaire 221 en matériau pyroélectrique est laissée apparente le long du premier bord S1 du support S à l'issue du dépôt de la bande 331 en matériau conducteur électrique. On ménage un espace selon la deuxième direction d'allongement X le long duquel la bande 221 en matériau pyroélectrique reste apparente.

A titre d'exemple, l'épaisseur de la troisième couche est comprise entre 100 et 2000 nanomètres, de préférence inférieure à 1000 nanomètres et plus précisément autour de 400 nm.

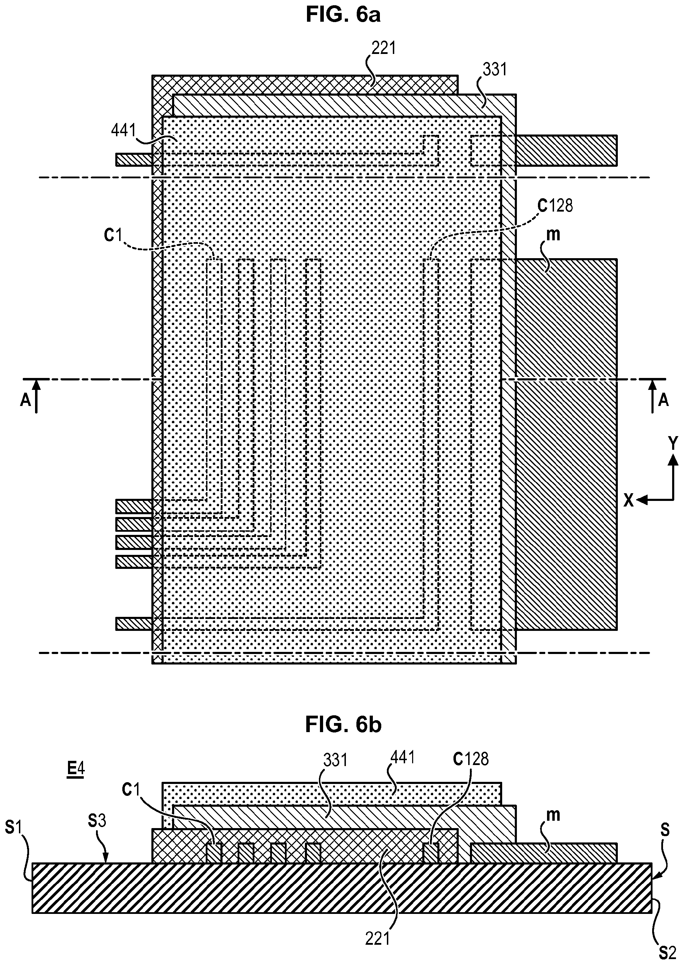

Une quatrième couche 400 (voir les **figures 6a** et **6b**) comprenant une bande 441 de largeur constante en matériau diélectrique en contact avec la bande 221 en matériau pyroélectrique de la deuxième couche 200 et la bande 331 en matériau métallique de la troisième couche 300.

La bande 441 rectangulaire en matériau diélectrique est en contact avec la troisième couche 300 et s'étend de manière à recouvrir les pistes de la première couche 100.

Cette quatrième couche 400 est avantageusement déposée par dépôt par voie liquide au moyen d'une filière à fente (« slot die ») en réalisant une bande continue dans la direction de déroulement du rouleau ou en réalisant de longues bandes dans le cas d'un mode feuille à feuille ou encore une juxtaposition de plusieurs bandes lorsque plusieurs matricés sont fabriquées en parallèle. De préférence, au cours de l'étape de dépôt les bandes s'étendent entre chaque ligne de découpe, la forme rectangulaire étant obtenue après découpe de la matrice.

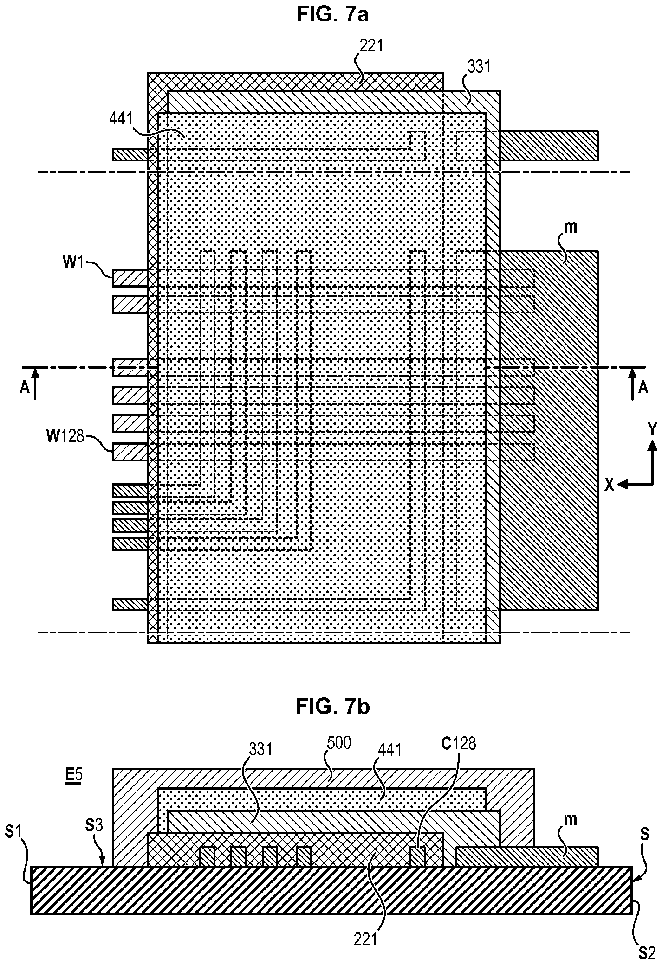

Une cinquième couche 500 (voir les **figures 7a** et **7b**) comprenant des pistes w₁, w₂, w₁₂₈ électriquement conductrices de chauffage disposées s'étendant depuis la bande de masse de la première couche vers le premier bord du support S de manière à recouvrir les pistes de la première couche. Ces pistes constituent les lignes de la matrice de pixels.

Les pistes w₁, w₂, w₁₂₈ de chauffage sont connectées à la bande de masse et ne vont pas au-delà des pattes p₁, p₁₂₈ de connexion. En outre, les pistes w₁, w₂, w₁₂₈ électriquement conductrices de chauffage sont connectées à l'électrode supérieure constituée par la troisième couche 300 ou à la bande de masse m. On note que dans le cas où la quatrième couche 400 déborde de la bande 331 de la troisième couche 300 le contact électrique est toujours assuré puisque les deux couches sont reliées à la même bande de masse.

A titre d'exemple, les pistes électriquement conductrices de chauffage sont en matériau métallique, typiquement de l'or, ou un métal moins cher (Argent, Aluminium ou Cuivre, etc.). L'épaisseur des bandes métalliques de chauffage est comprise entre 20 et 2000 nm, typiquement 500 à 1000 nm. Le choix du métal est peu important, l'essentiel étant d'avoir une résistance contrôlée.

Cette cinquième couche 500 est avantageusement déposée au moyen d'une impression par offset ou par héliogravure ou par dépôt sous vide de metal (PVD) associé à un moyen de photolithographie ou de structuration laser.

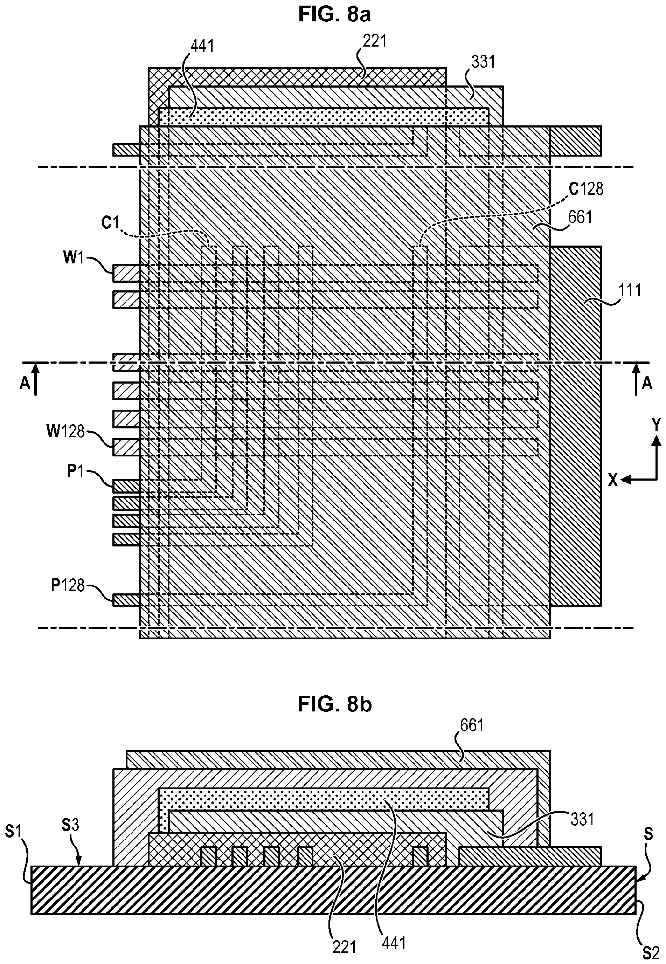

Une sixième couche 600 (voir les **figures 8a** et **8b**) comprenant une bande 661 de largeur constante de protection s'étendant selon l'axe X depuis la bande m de masse vers le premier bord s1 du support S.

La bande 661 rectangulaire de protection est telle qu'elle laisse apparente les pattes de connexion et les extrémités des pistes de chauffage w.

L'épaisseur de la bande rectangulaire de protection doit être optimisée pour protéger les couches au-dessous tout en laissant passer la chaleur.

Par exemple, l'épaisseur de la sixième couche est comprise entre 2 et 10 microns.

Cette sixième couche 600 est avantageusement déposée par voie liquide au moyen d'une filière à fente (« slot die » en réalisant une bande continue dans la direction de déroulement du rouleau ou en réalisant de longues bandes dans le cas d'un mode feuille à feuille ou encore une juxtaposition de plusieurs bandes lorsque plusieurs matrices sont fabriquées en parallèle. De préférence, au cours de l'étape de dépôt les bandes s'étendent entre chaque ligne de découpe, la forme rectangulaire étant obtenue après découpe du motif.

En outre, cette sixième couche 600 est avantageusement en matériau : DYMAX OC3021, DYMAX OC4122, PHC XH100, UVHC7300, UVL3.

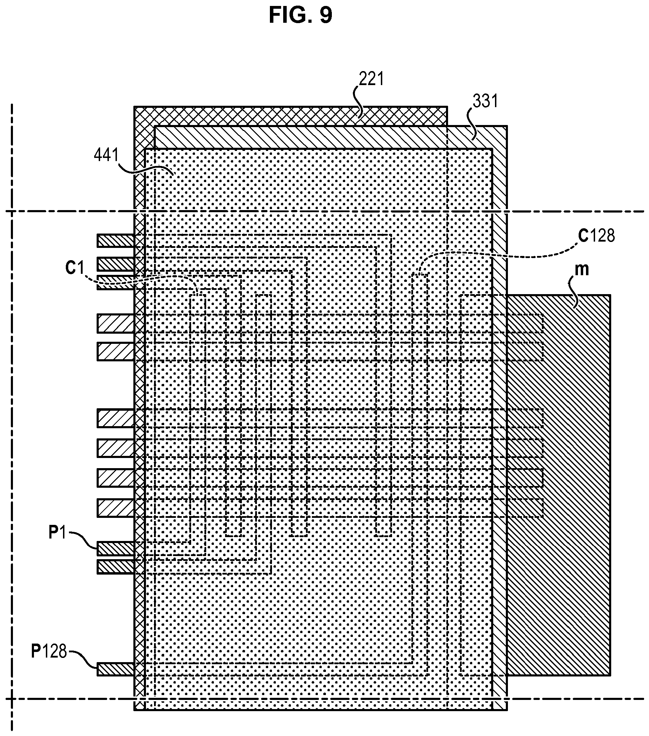

La **figure 9** illustre une matrice selon un deuxième mode de réalisation.

Ce deuxième mode de réalisation diffère du premier mode de réalisation par les dispositions des pattes p₁, p₁₂₈ de connexion de la première couche 100.

En particulier, les pistes et les pattes de connexion de la première couche forment une alternance de L et de L retournés de telle sorte que les pattes de connexions sont pour une partie au-dessus des pistes électriquement conductrices de chauffage de la cinquième couche et au-dessous des pistes électriquement conductrices de chauffage selon une direction Y du support S.

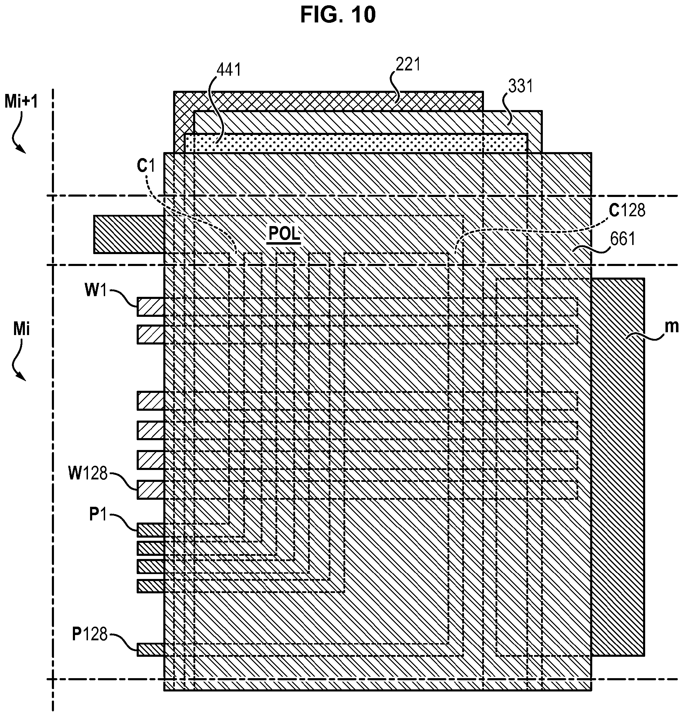

En relation avec la **figure 10** et de manière complémentaire au premier mode de réalisation, on peut prévoir que la première couche comprend en outre une bande POL de polarisation connectée aux pistes c₁, c₁₂₈. Cette bande POL de polarisation est dans ce cas imprimée entre deux matrices de pixels Mᵢ, Mᵢ₊₁.

En effet, pour que le matériau pyroélectrique acquière ses propriétés pyroélectriques il faut le polariser une bonne fois pour toute en le soumettant un champ électrique d'environ 100 à 120 volts par micron d'épaisseur du matériau pyroélectrique, par exemple 350 volts pour 3 microns. C'est par l'intermédiaire des pistes de la première couche que le matériau pyroélectrique de la deuxième couche 120 directement au-dessus des colonnes de la première couche 110 va être polarisé.

En outre, on prévoit dans ce mode de réalisation de la figure 10 des pistes de la première couche plus grandes que celles prévues dans le premier mode de réalisation, ceci pour permettre de connecter toutes les pistes.

Une fois le matériau pyroélectrique polarisé la zone comportant la bande de polarisation est découpée.

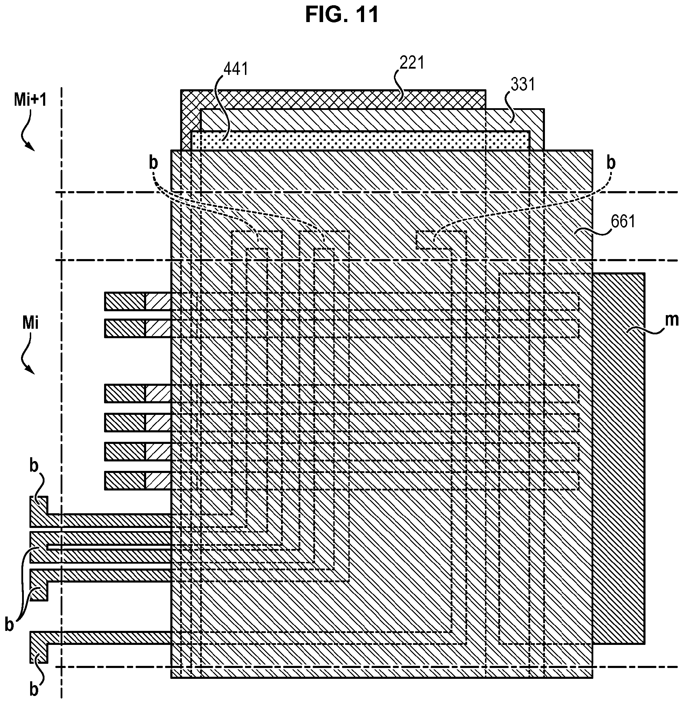

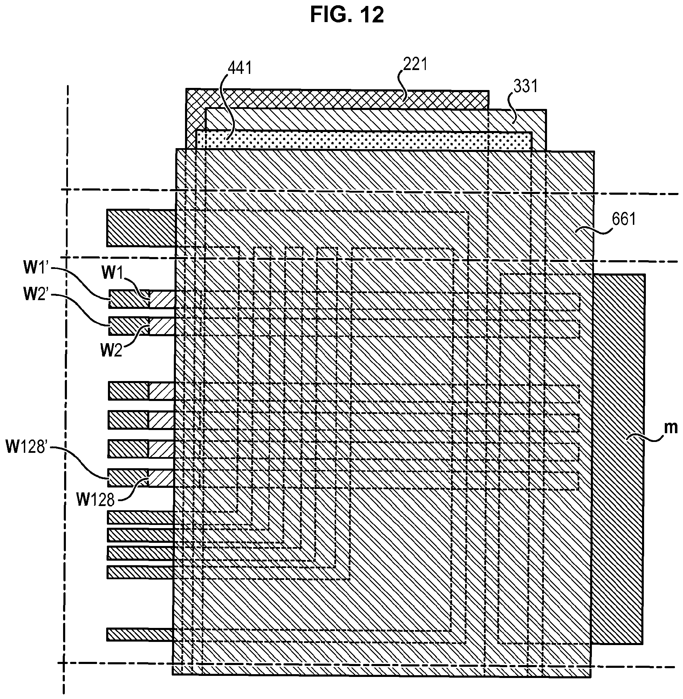

Toujours pour des questions de polarisation, en relation avec la **figure 11**, en variante, on prévoit que les pistes c₁, c₂ de la première couche forment un serpentin, les pistes étant connectés au moyen de bandelettes b métalliques dans des zones qui sont destinés à être découpées, une zone étant située entre deux matrices de pixels Mᵢ, Mᵢ₊₁ et l'autre zone, au-delà du bord de sortie (voir la ligne de découpe verticale à gauche sur la figure 12). On note que c'est dans cette zone que la bande de polarisation est imprimée.

Grâce à cette disposition de la figure 11, on peut alors contrôler l'ensemble des pistes avec une seule mesure de résistance et seulement deux pointes (au lieu de 128 + 1 dans la figure 10 (et impossible dans le premier mode de réalisation puisque l'autre côté ne sort pas).

En relation avec la **figure 12**, de manière complémentaire au premier mode de réalisation ou au second mode de réalisation, on prévoit que les pistes électriquement conductrices de chauffage se terminent au niveau du bord de sortie par des pavés conducteurs électriques w_{1'}, w_{2'}, w_{128'}. Les pavés conducteurs électriques sont du même matériau que celui utilisé pour la première couche et réalisés en même temps que celle-ci. Ainsi les lignes et les colonnes de la matrice sortent par le bord de sortie selon un seul et même métal ce qui facilite les connexions ultérieures. De plus la quatrième couche 400 peut être entièrement recouverte par la cinquième couche 500 pour la protéger. Ceci est particulièrement favorable dans le cas d'une couche en Argent pour prévenir sa sulfuration.

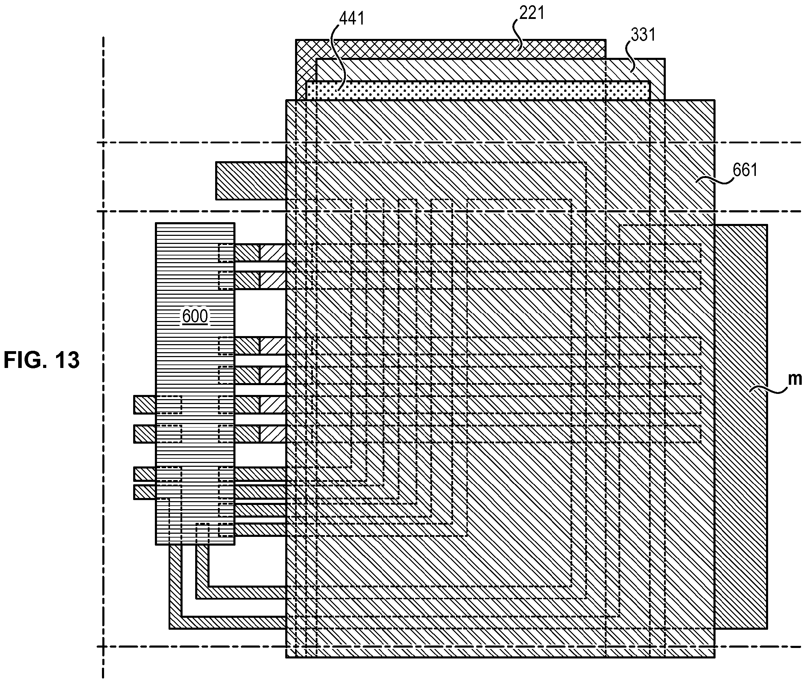

De tels pavés sont très avantageux lorsqu'il s'agira de connecter les lignes et les colonnes de la matrice à une puce 700 en silicium comme illustré sur la **figure 13**. La puce 700 est montée en flip-chip (puce retournée) c'est-à-dire la tête en bas avec ses plots de contact vers le bas en face des zones métalliques sortant par le bord de sortie.

La matrice ainsi décrite présente donc l'avantage d'être obtenue au moyen d'une technique d'impression à rouleaux comprenant plusieurs étages de dépôt par voie liquide « slot die ».

En outre, le fait de prévoir les pattes de connexion sur un des côtés du support S permet d'imprimer en flux continu des matrices de pixels sur un support S qui chemine d'étage à étage.

## Revendications

1. Procédé de fabrication d'une matrice de pixels d'un capteur de motif thermique, le procédé comprenant les étapes suivantes :
- fourniture d'un substrat (S) ;
- formation (E1) d'une première couche (100) en matériau électriquement conducteur, comprenant :
∘ un dépôt de pistes électriquement conductrices s'étendant selon une première direction d'allongement ;
∘ un dépôt de pattes de connexion, s'étendant depuis les pistes, selon une deuxième direction d'allongement, différente de la première direction d'allongement, et en se dirigeant vers un premier bord du substrat (S), dit bord de sortie ;
∘ un dépôt d'une bande de masse destinée à former la masse du capteur ;
- formation (E2) d'une deuxième couche (200) en matériau pyroélectrique recouvrant les pistes et laissant libre au moins une partie des pattes de connexion ;
- formation (E3) d'une troisième couche (300) en matériau électriquement conducteur déposée à cheval entre la couche de matériau pyroélectrique et la bande de masse sans recouvrir la partie libre des pattes de connexion ;
- formation (E4) d'une quatrième couche (400) en matériau diélectrique et s'étendant en face des pistes de la première couche ;
- dépôt (E5) d'une cinquième couche (500) comprenant des pistes électriquement conductrices chauffantes, lesdites pistes électriquement conductrices chauffantes s'étendant depuis la bande de masse vers le premier bord du substrat (S) de manière à être au-dessus des pistes de la première couche;
- dépôt (E6) d'une sixième couche (600) de protection ; procédé de fabrication **caractérisé en ce que** le dépôt de la deuxième couche (200) et/ou
le dépôt de la troisième couche et/ou le dépôt de la quatrième couche et/ou le dépôt de la sixième couche est mis en œuvre par dépôt par voie liquide au moyen d'une filière à fente.

2. Procédé de fabrication selon la revendication 1, dans lequel les couches sont formées successivement de la manière suivante : formation de la première couche, formation de la deuxième couche, formation de la troisième couche, formation de la quatrième couche, formation de la cinquième couche, formation de la sixième couche, la quatrième couche étant en contact avec la troisième couche.

3. Procédé de fabrication selon l'une des revendications précédentes, dans lequel la première couche (100) comprend en outre une bande de polarisation connectée à des colonnes du motif métallique de la première couche.

4. Procédé de fabrication selon l'une des revendications précédentes, dans lequel les pistes de la première couche forment un serpentin, les pistes étant connectées deux à deux au moyen de bandelettes métalliques.

5. Procédé de fabrication selon l'une des revendications précédentes, dans lequel la première couche comprend en outre des pavés métalliques destinés à être dans le prolongement des pistes électriquement conductrices chauffantes de la cinquième couche.

6. Procédé de fabrication selon l'une des revendications précédentes, dans lequel les pistes et les pattes de connexions de la première couche forment une pluralité de L imbriqués les uns dans les autres.

7. Procédé de fabrication selon l'une des revendications précédentes, à l'issue duquel les pattes de connexions sont toutes en-dessous des pistes électriquement conductrices chauffantes de la cinquième couche.

8. Procédé de fabrication selon l'une des revendications 1 à 5, dans lequel les pistes et les pattes de connexion de la première couche forment une alternance de L et de L retournés.

9. Procédé de fabrication selon l'une des revendications précédentes, dans lequel l'étape de formation (E3) de la troisième couche (300) est mise en œuvre de sorte à ménager un espace selon la deuxième direction d'allongement le long duquel la deuxième couche (200) en matériau pyroélectrique reste apparente.

10. Procédé de fabrication selon l'une des revendications précédentes, dans lequel au cours de l'étape de formation (E2) de la deuxième couche (200), on réalise une juxtaposition de plusieurs bandes de matériau pyroélectrique afin de réaliser plusieurs matrices de pixels en parallèle.

11. Matrice de pixels d'un capteur de motif thermique fabriquée par un procédé selon l'une des revendications précédentes.

12. Capteur de motif thermique comprenant une matrice de pixels selon la revendication précédente, la matrice étant typiquement passive ou active.

## Patentansprüche

1. Herstellungsverfahren einer Pixelmatrix eines thermischen Mustersensors, wobei das Verfahren die folgenden Schritte umfasst:
- Bereitstellung eines Substrats (S);
- Bildung (E1) einer ersten Schicht (100) aus elektrisch leitfähigem Material, umfassend:
o eine Beschichtung von elektrisch leitfähigen Bahnen, die sich in einer ersten Dehnungsrichtung erstrecken;
o eine Beschichtung von Verbindungslaschen, die sich von den Bahnen in einer zweiten Dehnungsrichtung erstrecken, die sich von der ersten Dehnungsrichtung unterscheidet und zu einer ersten Kante des Substrats (S), der Austrittskante, hin gerichtet sind;
o eine Beschichtung eines Massestreifens, der dazu bestimmt ist, die Masse des Sensors zu bilden;
- Bildung (E2) einer zweiten Schicht (200) aus pyroelektrischem Material, die die Bahnen bedeckt und mindestens einen Teil der Verbindungslaschen freilässt;
- Bildung (E3) einer dritten Schicht (300) aus elektrisch leitfähigem Material, die zwischen der Schicht aus pyroelektrischem Material und dem Massestreifen aufgebracht ist, ohne den freien Teil der Verbindungslaschen zu bedecken;
- Bildung (E4) einer vierten Schicht (400) aus dielektrischem Material, die sich gegenüber den Bahnen der ersten Schicht erstreckt;
- Beschichtung (E5) einer fünften Schicht (500), die elektrisch leitfähige Heizbahnen umfasst, wobei sich die elektrisch leitfähigen Heizbahnen vom Massestreifen in Richtung der ersten Kante des Substrats (S) erstrecken, um über den Bahnen der ersten Schicht zu liegen;
- Beschichtung (E6) einer sechsten Schutzschicht (600);
Herstellungsverfahren **dadurch gekennzeichnet, dass** die Beschichtung der zweiten Schicht (200) und/oder die Beschichtung der dritten Schicht und/oder die Beschichtung der vierten Schicht und/oder die Beschichtung der sechsten Schicht im Nassverfahren mittels einer Schlitzdüse erfolgt.

2. Herstellungsverfahren nach Anspruch 1, wobei die Schichten nacheinander wie folgt gebildet werden: Bilden der ersten Schicht, Bilden der zweiten Schicht, Bilden der dritten Schicht, Bilden der vierten Schicht, Bilden der fünften Schicht, Bilden der sechsten Schicht, wobei die vierte Schicht mit der dritten Schicht in Kontakt steht.

3. Herstellungsverfahren nach einem der vorhergehenden Ansprüche, wobei die erste Schicht (100) ferner einen Polarisationsstreifen umfasst, der mit Feldern des Metallmusters der ersten Schicht verbunden ist.

4. Herstellungsverfahren nach einem der vorhergehenden Ansprüche, wobei die Bahnen der ersten Schicht eine Spule bilden, wobei die Bahnen paarweise mittels Metallbändern verbunden sind.

5. Herstellungsverfahren nach einem der vorhergehenden Ansprüche, wobei die erste Schicht ferner Metallpads umfasst, die dazu bestimmt sind, in der Verlängerung der elektrisch leitfähigen Heizbahnen der fünften Schicht zu sein.

6. Herstellungsverfahren nach einem der vorhergehenden Ansprüche, wobei die Bahnen und die Verbindungslaschen der ersten Schicht mehrere ineinander verschachtelte L bilden.

7. Herstellungsverfahren nach einem der vorhergehenden Ansprüche, nach dessen Beendigung sich die Verbindungslaschen alle unterhalb der elektrisch leitenden Heizbahnen der fünften Schicht befinden.

8. Herstellungsverfahren nach einem der Ansprüche 1 bis 5, wobei die Bahnen und die Verbindungslaschen der ersten Schicht eine Abwechslung von L und umgedrehten L bilden.

9. Herstellungsverfahren nach einem der vorhergehenden Ansprüche, wobei der Schritt des Bildens (E3) der dritten Schicht (300) so ausgeführt wird, dass ein Raum in der zweiten Dehnungsrichtung freigestellt ist, entlang dem die zweite Schicht (200) aus pyroelektrischem Material sichtbar bleibt.

10. Herstellungsverfahren nach einem der vorhergehenden Ansprüche, wobei während des Schritts des Bildens (E2) der zweiten Schicht (200) eine Aneinanderreihung mehrerer Streifen pyroelektrischen Materials realisiert wird, um mehrere Pixelmatrizen parallel zu realisieren.

11. Pixelmatrix eines thermischen Mustersensors, hergestellt nach einem Verfahren nach einem der vorhergehenden Ansprüche.

12. Thermischer Mustersensor, eine Pixelmatrix nach dem vorhergehenden Anspruch umfassend, wobei die Matrix vom Typ passiv oder aktiv ist.

## Claims

1. Method for manufacturing a pixel array of a thermal pattern sensor, the method comprising the following steps:
- providing a substrate (S);
- forming (E1) a first layer (100) of electrically conductive material, comprising:
• a deposit of electrically conductive tracks extending in a first direction of elongation;
• a deposit of connection lugs, extending from the tracks, in a second direction of elongation, different from the first direction of elongation, and heading towards a first edge of the substrate (S), called the exit edge;
• a deposit of a ground strip intended to form the ground of the sensor;
- forming (E2) a second layer (200) of pyroelectric material covering the tracks and leaving at least one part of the connection lugs free;
- forming (E3) a third layer (300) of electrically conductive material deposited astride the layer of pyroelectric material and the ground strip without covering the free part of the connection lugs;
- forming (E4) a fourth layer (400) of dielectric material and extending across the tracks of the first layer;
- depositing (E5) a fifth layer (500) comprising electrically conductive heater tracks, said electrically conductive heater tracks extending from the ground strip towards the first edge of the substrate (S) so as to be above the tracks of the first layer;
- depositing (E6) a sixth layer (600) of protection;
manufacturing method **characterised in that** the deposit of the second layer (200) and/or the deposit of the third layer and/or the deposit of the fourth layer and/or the deposit of the sixth layer is implemented by liquid deposit by means of a slot die.

2. Manufacturing method according to claim 1, wherein the layers are formed successively in the following way: formation of the first layer, formation of the second layer, formation of the third layer, formation of the fourth layer, formation of the fifth layer, formation of the sixth layer, the fourth layer being in contact with the third layer.

3. Manufacturing method according to one of the preceding claims, wherein the first layer (100) also comprises a polarisation strip connected to columns of the metal pattern of the first layer.

4. Manufacturing method according to one of the preceding claims, wherein the tracks of the first layer form a coil, the tracks being connected in pairs by means of metal strips.

5. Manufacturing method according to one of the preceding claims, wherein the first layer also comprises metal pads intended to be in the extension of the electrically conductive heater tracks of the fifth layer.

6. Manufacturing method according to one of the preceding claims, wherein the tracks and the connection lugs of the first layer form a plurality of interlocking Ls.

7. Manufacturing method according to one of the preceding claims, at the end of which the connection lugs are all below the electrically conductive heater tracks of the fifth layer.

8. Manufacturing method according to one of claims 1 to 5, wherein the tracks and the connection lugs of the first layer form an alternation of Ls and inverted Ls.

9. Manufacturing method according to one of the preceding claims, wherein the step of forming (E3) the third layer (300) is implemented in such a way as to create a space in the second direction of elongation along which the second layer (200) of pyroelectric material remains visible.

10. Manufacturing method according to one of the preceding claims, wherein during the step of forming (E2) the second layer (200), a juxtaposition of several strips of pyroelectric material is made in order to produce several pixel arrays in parallel.

11. Pixel array of a thermal pattern sensor manufactured by a method according to one of the preceding claims.

12. Thermal pattern sensor comprising a pixel array according to the preceding claim, the array being typically passive or active.
